Europäisches Patentamt

European Patent Office

Office européen des brevets

(11) Publication number: **0 170 922**
**A1**

# EUROPEAN PATENT APPLICATION

(21) Application number: 85108586.0

(22) Date of filing: **10.07.85**

(51) Int. Cl.⁴: **A 41 B 13/02**

(30) Priority: **20.07.84 JP 150790/84**

(43) Date of publication of application: **12.02.86**
**Bulletin 86/7**

(84) Designated Contracting States: **DE FR GB IT**

(71) Applicant: **Kao Corporation, 14-10, Nihonbashi Kayabacho 1 chome, Chuo-Ku Tokyo 103 (JP)**

(72) Inventor: **Koizumi, Yasushi, 1192-9 Kishikawada-machi, Utsunomiya-shi Tochigi-ken (JP)**

(74) Representative: **Patentanwälte Grünecker, Kinkeldey, Stockmair & Partner, Maximilianstrasse 58, D-8000 München 22 (DE)**

(54) **Method for attaching plastic strip to sheet.**

(57) A method for manufacturing a disposable diaper containing elastic strips which comprises continuously providing a running length of a sheet of material (1), continuously providing an elastic strip (4) in a stretched condition, intermittently applying a release agent (18) to selected portions of said stretched elastic strip, feeding said stretched elastic strip onto a portion of said sheet (1), said elastic strip being fixed to the sheet in the areas which are free of the release agent, and cutting the elastic strip in areas having release agents applied thereto together with said sheet.

S P E C I F I C A T I O N

BACKGROUND AND SUMMARY OF THE INVENTION

The present invention relates to a method for attaching an elastic strip to a sheet and, more particularly, to such a method suitable for attaching elastic strips to a disposable diaper along its side flaps.

To obtain an optimal fit around a baby's crotch, a disposable diaper has already been proposed in which the side flaps are provided with elastic. For such disposable diapers, providing elastic strips along the full length on both side flaps would make the elastic portions of the diaper too long. To avoid this problem, it is preferred to attach an appropriate length of the elastic strips merely around the crotch

2    0170922

area.

To accomplish this, there has already been proposed a method for attaching elastic strips to a sheet in which the elastic strips are maintained stretched in the area of the crotch but non-stretched in the other areas.  See for example, Japanese Patent Publication 26219/1982, and Japanese Unexamined Disclosures 181865/1982 and 69072/1984.

The Patent Publication 26219/1982 discloses a method by which stretched elastic strips are intermittently applied with adhesives and fed onto a sheet.  However, this method is accompanied by the problem of adhesive stringiness or wedding.

More specifically, assume that the treatment is done by this method at a rate of 300 diapers/min., with the velocity of the sheet passing through the apparatus being 150 m/min. when each diaper has a length of 50 cm.  Correspondingly, the stretched elastic strips also will have the same velocity of 150 m/min. and the adhesives should be intermittently applied onto these elastic strips fed at this velocity.  As to the adhesive, the hot melt type will be the most efficient and feasible with the use of, for example, the hot melt applicator device supplied from Nordson Co., Ltd., or

- 2 -

0170922

the like.  The hot melt will be intermittently discharged through a discharge nozzle at a relatively high viscosity of approximately 1000 to 5000 cps.  The intermittently discharge of the adhesive tends to cause so-called stringiness or wedding which may, in turn, cause each sport of application to overrun a predetermined length or the elastic strips to be fixed to the sheet, out of a predetermined position.

The Unexamined Disclosure 181865/1982 describes a method by which an adhesive is continuously applied onto a sheet and, after the absorbent, having lugs at the opposite ends has been fed, elastic strips are introduced.  However, this method results in a relatively wide absorbent, since the absorbent requires lugs.

Finally, Unexamined Disclosure 69072/1984 describes a method by which elastic strips are applied with adhesives while a sheet is applied with release agents.  However, it is necessary for this method to apply the release agents in a relatively large width or to register positions of the release agents and the elastic strips with respect to each other by a high level of control.

According to the present invention, the above-mentioned problems are solved by incorporating and appropriately combining the step of continuously applying an adhesive onto a sheet with the step of applying release agents onto an elastic strip in areas along which said elastic strip is not to be fixed to said sheet.

Thus, the present invention provides a method for attaching an elastic strip to a sheet, said method comprising the steps of:

(a) continuously feeding a sheet;

(b) continuously applying an adhesive onto said sheet;

(c) introducing an elastic strip under a stretched condition;

(d) intermittently applying a release agent to selected portions of said elastic strip being fed under said stretched condition;

(e) applying said elastic strip thus stretched and treated with release agents to a portion of said sheet having the adhesive applied thereto so that said elastic strip is fixed to said sheet along the areas having no release agent thereon; and

(f) cutting said elastic strip in areas having release agents applied thereon together with said sheet.

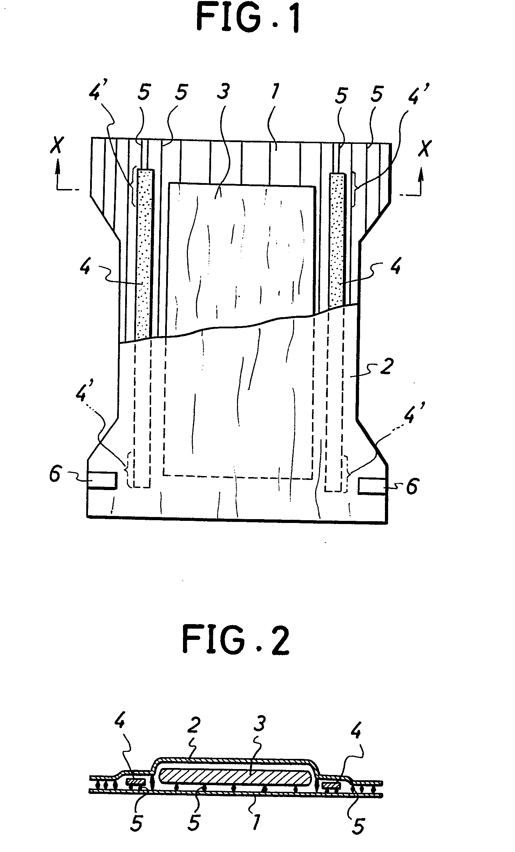

A more detailed understanding of the present invention may be had from the following description of a preferred embodiment of the present invention, which is given by way of example only by referring to Figs. 1 and 2 of the accompanying drawings.

## BRIEF DESCRIPTION OF THE DRAWINGS

The present invention will become more fully understood from the detailed description given hereinbelow and the accompanying drawings which are given by way of illustration only, and thus are not limitative of the present invention, and wherein:

Fig. 1 is a schematic plan view showing, partially broken away, the disposable diaper manufactured using the method of the present invention;

Fig. 2 is a section taken along a line X - X of Fig. 1; and

Fig. 3 is a side view schematically illustrating a manner in which the present invention is embodied and important elements of an apparatus for executing the present invention.

DETAILED DESCRIPTION OF THE DRAWINGS

A brief description will first be made with respect to the manufacture of the disposable diaper using the method according to the present invention and as illustrated by Figs. 1 and 2.

Fig. 1 is a schematic plan view showing, partially broken away, the disposable diaper manufactured using the method of the present invention, and Fig. 2 is a section taken along line X - X of Fig. 1, wherein reference numeral 1 designates a first sheet made of a water-impermeable material, reference numeral 2 designates a second sheet made of a water-permeable material, reference numeral 3 designates an absorbent material, reference numeral 4 designates an elastic strip and reference numerals 5,5,5 designate continuous beads of adhesive applied onto the first sheet. The absorbent 3 is centrally placed on the first sheet and a pair of elastic strips 4,4 are laterally disposed along both sides of said absorbent 3. Such an assembly is covered with and fixed to the second sheet through the adhesives 5 so as to form a desired disposable diaper.

More specifically, both the absorbent 3 and the

- 6 -

elastic strips 4,4 are fixed along their central areas to the first sheet 1 through the adhesives 5 as seen in Fig. 1. However, the respective elastic strips 4,4 are not fixed along their opposite side edges 4',4' to the first sheet 1. Since these side edges 4',4' are applied with release agents, as will be described later, the adhesives 5 are not effective with respect to said side edges. Thus, only the remainder, central areas are fixed in a stretched condition onto the first sheet through the adhesives 5. Reference numerals 6,6 designate fastener tapes used for attaching the diaper. It should be understood that the adhesive 5 may be applied substantially in a continuous manner. Accordingly, the expression used herein "an adhesive is continuously applied" should be understood to include that an adhesive is applied in the form of a plurality of substantially continuous beads. Shapes and materials for the first and second sheets, the absorbent and the elastic strips as well as the type of adhesive may be selected from conventional materials known in the art.

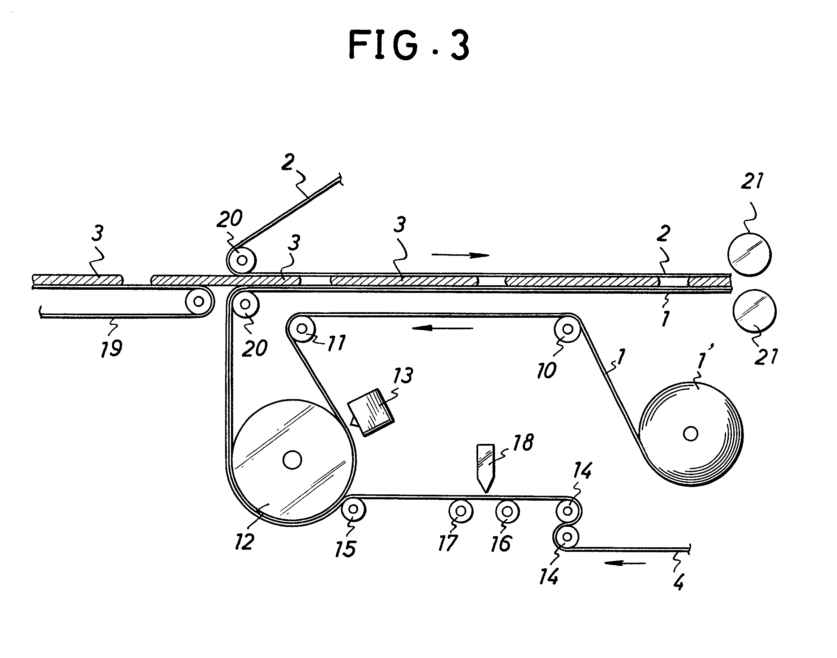

The steps for manufacturing the above-mentioned disposable diaper, and particularly the method for attaching the elastic strips onto the first sheet, will now be discussed with reference to Fig. 3.

Fig. 3 is a side view schematically illustrating a manner in which the present invention is embodied and important elements of an apparatus for the execution of the present invention. Reference numerals 10,11 designate guide rollers for the first sheet, reference numeral 12 designates a cooling drum, and reference numeral 13 designates an adhesive applicator. The first sheet 1 is fed from a sheet roll 1' through the guide rollers 10,11 to the cooling drum 12 upon which the first sheet 1 is fixed onto the second sheet 2 through the adhesives. The adhesive applicator 13 is so arranged and located that the adhesives are applied onto the first sheet 1 in a plurality of lines (4 to 32 lines, 17 lines in the embodiment illustrated) each comprising substantially continuous beads as seen in Fig. 1 and, in the case illustrated, the adhesives 5 (not shown in Fig. 3) may be applied onto the first sheet 1 immediately after said sheet 1 has been fed to the cooling drum 12.

Reference numerals 14,14 designate the elastic strip feed rollers. Reference numeral 15 designates an elastic strip stretching roller. Reference numerals 16,17 designate guide rollers for the elastic strips 4, and reference numeral 18 designates a release agent applicator. The elastic strips 4, fed through the elastic strip feed rollers 14,14 are stretched between said elastic strip feed rollers 14,14 and the elastic strip stretching roller 15 by varying velocities of these members. The elastic strip is then fed in a stretched condition through the guide rollers 16,17 and the stretching roller 15 and fed while still in the stretched condition onto the first sheet 1 passing the cooling drum 12 so that the elastic strips 4 are fixed onto said first sheet 1 through the adhesive 5. The release agent applicator 18 serves for intermittent application of release agents (not shown) onto the elastic strips 4 being fed in the stretched condition and located between the elastic strip feed rollers 14,14 and the elastic strip stretching roller 15.

Reference numeral 19 designates a conveyor adapted for continuously feeding the absorbents at given intervals and reference numerals 20,20 designate press rollers between which the first sheet 1 having the elastic strips fixed thereto and fed from the cooling drum 12 is adhesively fixed to the second sheet separately fed with the interposition of said absorbent 3.

Reference numerals 21,21 designate roll-type cutters. The first sheet 1 and the second sheet 2 have been laid upon and adhesively fixed to each other with the interposition of the adsorbent 3 as well as a pair of the elastic strips 4 (as shown in Fig. 3) by passing the composite through said press rollers 20,20 into a laminate assembly which is then cut by said roll-type cutter in the areas where the elastic strips 4 have release agent applied thereto.

As seen in Fig. 1, a pair of the elastic strips 4 are fed so that they are adhesively fixed onto the first sheet 1 in parallel to each other with a given spacing while the absorbent 3 is fed so as to be placed between said pair of elastic strips. Release agents are applied onto the elastic strips along the areas 4',4' (See Fig. 1) where the latter are not to be adhesively fixed to the sheet 1. After the assembly has been cut along the areas having the release agent applied thereon, the disposable diaper, as illustrated by Fig. 1, is formed. Although not shown, there are separately provided cutters adapted to cut both sides of said laminate assembly before the assembly is cut by the cutter 21,21 to form the diaper of Fig. 1.

The method for attaching the elastic strips onto the sheet will be realized in the following manner, when the above-described apparatus is employed.

- The first sheet 1 is fed from the sheet roll 1' through the guide rollers 10,11 to the cooling drum 12 while the adhesive applicator 13 continuously applies the hot melt adhesives 5 onto said sheet 1 in a plurality of lines each comprising substantially continuous beads as shown in Figs. 1 and 2.

On the other hand, a pair of the elastic strips 4 spaced from each other at a given distance are fed by the elastic strip feed rollers 14,14 through the guide rollers 16,17 and the stretching roller 15, and the r.p.m. of said feed rollers 14,14 is adjusted to be lower than that of the stretching roller 15 so that the elastic strips 4 may be placed in a stretched condition. A release agent applicator 18 intermittently applies release agents onto the elastic strips 4 which are being conveyed in the stretched condition. As a result, the pair of elastic strips 4 are intermittently applied with release agents and conveyed in a stretched condition from the stretching roller 15 to the cooling drum 12 upon which the elastic strips are applied to the first sheet 1 in the areas which have been treated with the adhesives 5. Thus, each of the

- 11 -

elastic strips 4 is not adhesively fixed on sheet 1 in the areas having the release agents applied thereon but are adhesively fixed in the areas where no release agent has been applied by the adhesives 5. Although two lines of adhesive are provided for each elastic strip, in the embodiment shown, usually 1 to 4 lines are provided for each elastic strip, in the stretched condition. The adhesives are solidified by the cooling drum 12 and thus the elastic strips are integrated with the first sheet 1.

The first sheet 1, having the elastic strips 4 adhesively fixed thereto, is fed through the press rollers 20,20 where the absorbent 3 fed by the conveyor 19 is incorporated while the separately supplied second sheet 2 is adhesively fixed onto this assembly. The newly formed assembly then leaves the press rollers 20,20.

After the completion of the above steps, a laminate assembly is formed in which the absorbent 3 and the pair of elastic strips 4 are sandwiched between the first sheet 1 and the second sheet 2 as seen in Fig. 3.

Thereafter, the laminate assembly is transferred to the step of cutting in which the laminate assembly is cut by the cutter 21,21 along the areas of the elastic strips 4 having the release agents applied thereon. Prior to this step of cutting, both side sections of said laminate assembly are cut away (not shown) so as to form the diaper as shown in Fig. 1.

As a result, the areas 4' of the elastic strips 4, having the release agent applied thereon are restored to their initial non-stretched conditions. Thus, only the portions of the elastic strips located at the side flaps of the diaper are maintained stretched and adhesively fixed onto the first sheet to provide the diaper with the configuration as shown in Figs. 1 and 2.

In the method for attaching the elastic strip onto the sheet in accordance with the present invention, the step of continuously applying the adhesive onto the sheet and the step of applying release agents onto the elastic strip in the areas where the elastic strip is not to be adhesively fixed onto the sheet, are incorporated so that the elastic strip previously applied with the release agents along the areas not be adhesively fixed onto the sheet in a separate step may be adhesively fixed onto said sheet being continuously applied with adhesive. By employing the method according to the present invention particularly for attaching the elastic strips to the disposable diaper, the problem of the adhesive's stringiness or wedding is avoided, it is unnecessary to use the absorbent having lugs and thereby an inconveniently large width of the adsorbent is avoided. Also, a high level of quality control is not required for precisely registering positions of the release agents and the elastic strips, and various problems relating to application of the release agents onto the sheet are also avoided, permitting the disposable diapers to be continuously manufactured with high efficiency.

The invention being thus described, it will be obvious that the same may be varied in many ways. Such variations are not to be regarded as a departure from the spirit and scope of the invention, and all such modifications as would be obvious to one skilled in the art are intended to be included within the scope of the following claims.

0170922

WHAT IS CLAIMED IS:

1. A method for manufacturing a disposable diaper containing elastic strips which comprises
continuously providing a running length of a sheet of material,
continuously providing an elastic strip in a stretched condition,
intermittently applying a release agent to selected portions of said stretched elastic strip,
feeding said stretched elastic strip onto a portion of said sheet, said elastic strip being fixed to the sheet in the areas which are free of the release agent, and
cutting the elastic strip in areas having release agents applied thereto together with said sheet.

2. The method of claim 1 wherein an absorbent material is applied to the sheet and adhesively affixed thereto to from a composite and a cover sheet is applied to said composite.

3. The method of claim 2 wherein the absorbent material is positioned in the middle of the sheet of

- 15 -

material and elastic strips are adhered to said sheet on both sides of said absorbent material.

4. The method of claim 1 wherein the adhesive is applied in a plurality of longitudinally extending strips.

5. The method of claim 1 wherein the elastic strip is stretched by conveying it over rolls rotating at different speeds.

# FIG.1

# FIG.2

# FIG·3

## European Patent Office

**EUROPEAN SEARCH REPORT**

### DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y,D | US-A-4 353 762 (F.J. BOUDA) * Column 3, lines 3-54; column 4, lines 9-18; claims; figures * & JP - A - 59 69 072 --- | 1 | A 41 B 13/02 |
| Y | US-A-4 333 782 (JOHNSON & JOHNSON BABY PRODUCTS CO.) * Column 11, last paragraph; column 12, lines 1,2; figures 11,12 * | 1 | |
| A | --- | 2-4 | |
| A,D | DE-A-2 649 948 (THE PROCTER & GAMBLE CO.) * Page 27, paragraph 2; page 28, paragraph 1; page 30, paragraph 3; page 31, paragraph 1; figures 1,3 * & JP - A - 57 26 219 ----- | 5 | |

**TECHNICAL FIELDS SEARCHED (Int. Cl.4)**

A 41 B

The present search report has been drawn up for all claims

| Place of search | Date of completion of the search | Examiner |
|---|---|---|
| THE HAGUE | 31-10-1985 | GARNIER F.M.A.C. |